(19) **Europäisches Patentamt / European Patent Office / Office européen des brevets**

(11) **EP 4 786 576 A1**

(12) # EUROPEAN PATENT APPLICATION

(43) Date of publication:
**05.08.2026 Bulletin 2026/32**

(21) Application number: **25305155.1**

(22) Date of filing: **03.02.2025**

(51) International Patent Classification (IPC):
*C12N 1/18* $^{(2026.01)}$   *C12P 7/06* $^{(2006.01)}$
*C12R 1/865* $^{(2006.01)}$

(52) Cooperative Patent Classification (CPC):
**C12N 1/185; C12P 7/06;** C12R 2001/865

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **VIB VZW**
  **9052 Gent (BE)**
• **Katholieke Universiteit Leuven**
  **3000 Leuven (BE)**
• **Lesaffre et Compagnie**
  **75001 Paris (FR)**

(72) Inventors:
• **VERSTREPEN, Kevin**
  **3001 Heverlee-Leuven (BE)**
• **STEENSELS, Jan**
  **3001 Heverlee-Leuven (BE)**
• **SPAEPEN, Stijn**
  **3001 Heverlee-Leuven (BE)**
• **SERPA MULLER, Viviane**
  **59700 MARCQ-EN-BAROEUL (FR)**
• **FROMANGER, Romain**
  **59700 MARCQ-EN-BAROEUL (FR)**

(74) Representative: **Ipsilon**
  **Le Contemporain**
  **50 Chemin de la Bruyère**
  **69574 Dardilly Cedex (FR)**

(54) **YEAST STRAINS FOR HIGHER ETHANOL CONTENT IN INDUSTRIALLY RELEVANT CONDITIONS**

(57)   The present invention relates to a fermenting organism which is a *Saccharomyces cerevisiae* strain selected from:

a. *Saccharomyces cerevisiae strain H147, which was deposited on December 20, 2024 at the CNCM under Number I-6168*; and

b. *Saccharomyces cerevisiae strain H12, which was deposited on December 20, 2024 at the CNCM under Number I-6167,*
   Or a derivative thereof.

EP 4 786 576 A1

## Description

This application contains a reference to two deposits of biological material, which are incorporated herein by reference.

## FIELD OF THE INVENTION

The present invention relates to improved fermenting organisms or derivatives thereof, as well as compositions thereof and process for producing ethanol using the same.

## BACKGROUND OF THE INVENTION

Bioethanol has been identified as the mostly used biofuel worldwide since it significantly contributes to the reduction of crude oil consumption and environmental pollution. It can be produced from various types of feedstocks such as sucrose, starch, lignocellulosic and algal biomass through fermentation process by microorganisms. Compared to other types of microorganisms, yeasts, especially *Saccharomyces cerevisiae,* are the common microbes employed in ethanol production due to their high ethanol productivity, high ethanol tolerance and ability of fermenting a wide range of sugars.

However, there are some challenges in yeast fermentation which inhibit ethanol production such as high temperature, high ethanol concentration and the ability to ferment pentose sugars. Various types of yeast strains have been used in fermentation for ethanol production including hybrid, recombinant and wild-type yeasts. Yeasts can directly ferment simple sugars into ethanol while other types of feedstocks must be converted to fermentable sugars before it can be fermented to ethanol. The common processes involved in ethanol production are pretreatment, hydrolysis and fermentation. Production of bioethanol during fermentation depends on several factors such as temperature, sugar concentration, pH, fermentation time, agitation rate, and inoculum size (Mohd Azhar SH, Abdulla R, Jambo SA, Marbawi H, Gansau JA, Mohd Faik AA, Rodrigues KF. Yeasts in sustainable bioethanol production: A review. Biochem Biophys Rep. 2017 Mar 6;10:52-61. doi: 10.1016/j.bbrep.2017.03.003. PMID: 29114570; PMCID: PMC5637245).

Yeast of the genus *Saccharomyces* exhibits many of the characteristics required for production of ethanol. In particular, strains of *Saccharomyces cerevisiae* are widely used for the production of ethanol in the fuel ethanol industry. Strains of *Saccharomyces cerevisiae* that are widely used in the fuel ethanol industry can produce high yields of ethanol under fermentation conditions, for example when fermenting corn mash. An example of such a strain is the yeast present in the commercially available ethanol yeast product called Ethanol Red® (deposited on September 4, 2008 under Accession number I-4071 at CNCM) (da Silva Fernandes F, de Souza ÉS, Carneiro LM, Alves Silva JP, de Souza JVB, da Silva Batista J. Current Ethanol Production Requirements for the Yeast Saccharomyces cerevisiae. Int J Microbiol. 2022 Aug 13;2022:7878830. doi: 10.1155/2022/7878830. PMID: 35996633; PMCID: PMC9392646).

Although the strains of *Saccharomyces cerevisiae* currently used in the fuel ethanol industry are well suited to ethanol production, there is an increasing need for improvements in the efficiency of ethanol production owing to the increased demand for ethanol as a fuel. Further, in regions and markets where GMO (genetically modified organism) yeast strains are restricted or prohibited, ethanol producers face additional challenges in achieving high fermentation performance. This constraint means that ethanol producers must rely on non-GMO approaches to generate new yeast variants that vastly outperform the current commercial strains.

Documents CN118146965 and CN117844664 both pertain to yeast strains obtained by ARTP mutagenesis technology and high-throughput screening, wherein the strains have excellent tolerance and high ethanol production ability.

Document WO2024/123691 pertains to modified yeasts with disrupted genes that produce an increased amount of ethanol, and, in some cases, a decreased amount of acetate compared to otherwise identical parental cells. Such yeast being useful for large-scale ethanol production from starch substrates with high dissolved solids.

Document WO2024/064901 pertains to yeasts strains of *Saccharomyces* capable of improving the efficiency of ethanol production in industrial scale fermentation.

Document WO2016/174349 pertains to methods for selecting a yeast strain having improved capability for fermenting a pentose, advantageously xylose, in the presence of organic acid, advantageously acetic acid, in non-dissociated form.

Document WO2015/121595 pertains to methods for obtaining a strain capable of efficiently propagating in a low nutritive potential medium, capable of metabolizing pentoses and of resisting fermentation inhibitors.

Document WO2022/216622 relates to yeast strains Y1912, Y1913, Y1914, Y1919, Y1923, Y1927, Y1929 and derivatives thereof, as well as compositions comprising the yeast strains for use in ethanol manufacture.

However, there is still a need for new robust yeast strains of *Saccharomyces* capable of improving the efficiency of ethanol production during industrial scale fermentation while being economical and good for the environment.

## SUMMARY OF THE INVENTION

The present inventors have now found a way to improve the efficiency of ethanol production in industrial scale fermentation.

A first object of the invention thus relates to a fermenting organism which is a *Saccharomyces cerevisiae* strain selected from :

the *Saccharomyces cerevisiae* strain which was deposited on December 20, 2024 at the CNCM (Collection Nationale de Cultures de Microorganismes, Institut Pasteur, 25 rue du Docteur Roux, 75724 Paris Cedex 15), under accession number I-6168 under the conditions of the Budapest Treaty; and

the *Saccharomyces cerevisiae* strain which was deposited on December 20, 2024 at the CNCM, under accession number I-6167 under the conditions of the Budapest Treaty.

A second object of the invention pertains to a derivative of the fermenting organism as described herein.

A third object of the invention pertains to a composition comprising the fermenting organism or the derivative as described herein, and one or more components selected from the group consisting of surfactants, emulsifiers, gums, swelling agents, protectants, antioxidants and other processing aids.

A fourth object of the invention pertains to the process for producing ethanol from a biomass comprising contacting the biomass with the fermenting organism or the derivative or the composition as described herein.

A fifth object of the invention pertains to the derivative as described herein wherein it is a hybrid produced by culturing a first yeast strain with the *Saccharomyces cerevisiae* strain of the invention under conditions which permit combining of DNA between the first yeast strain and the *Saccharomyces cerevisiae* of the invention.

A sixth object of the invention pertains to the derivative as described herein wherein it is a mutant produced by spontaneous mutation of the genome of the fermenting organism as described herein or induced by mutagenesis.

Finally, one last object of the invention pertains to the derivative as described herein wherein it is a recombinant produced by genetically modifying the fermenting organism as described herein to express a heterologous enzyme from the group consisting of alphaamylase, glucoamylase xylose isomerase, xylose reductase, xylitol dehydrogenase, D-xylulokinase, arabinose isomerase, ribulokinase, a ribulose 5 phosphate epimerase, and combinations thereof.

## DETAILED DESCRIPTION OF THE INVENTION

### Definitions

In the following, terms as used herein are defined in their meaning.

The use of the articles "a", "an", and "the" in both the description and claims are to be construed to cover both the singular and the plural, unless otherwise indicated herein or clearly contradicted by context.

The terms "comprising", "having", "being of", "including", and "containing" are to be construed as open terms (i.e., meaning "including but not limited to") unless otherwise noted. Additionally, whenever "comprising" or another open-ended term is used in an embodiment, it is to be understood that the same embodiment can be more narrowly claimed using the intermediate term "consisting essentially of" or the closed term "consisting of".

The term "about" or "ca." has herein the meaning that the following value may vary for $\pm$ 10%, preferably $\pm$ 5%, more preferably $\pm$ 2%, even more preferably $\pm$ 1%.

Unless otherwise defined, "%" has herein the meaning of volume or weight percent (v/v% or w/w%), also referred to as volume by volume percent (v/v%) or weight by weight percent (w/w%) respectively.

The term "yeast", in the singular or in the plural, is a generic term referring to eukaryotic microorganisms capable of causing a fermentation of organic materials. Yeasts are commonly used, whether by individuals or by the agri-food or pharmaceutical industry. For example, they may be used in the manufacture of wine, beer, or leavened doughs (for example breads). Among yeasts, mention will be made, in a non-exhaustive manner, of the genus *Saccharomyces,* including but not limited to the species S. *cerevisiae.*

The expression "yeast strain" refers to a relatively homogeneous population of yeast cells. A yeast strain is obtained from the isolation of a clone. A clone gives birth to a population of cells obtained from one single yeast cell. A yeast is generally produced based on the multiplication of a strain cultured in a medium comprising a set of raw materials. In an industrial environment, based on a given starting amount of yeast, it is possible to produce several tons at the output.

The expression "derived yeast strain" or "derivative of a yeast strain" refers to a yeast strain derived by one or more crosses and/or by mutation and/or by genetic transformation.

A yeast strain derived by crossing can be obtained by cross-breeding of a yeast strain according to the invention with the same yeast strain, with another yeast strain according to the invention, or with any other yeast strain (provided that it can be crossed, by which the person skilled in the art understands that it is homozygous for the MAT locus and that there is no mutation in the STE genes).

A yeast strain derived through mutation can be a yeast strain which has undergone at least one spontaneous mutation in the genome thereof or at least one mutation induced by mutagenesis. The mutation(s) of a derived strain may be silent or not.

A yeast strain derived by genetic transformation is a yeast strain in which a DNA sequence was introduced that is preferably supplied by a plasmid or incorporated directly into the genome. For example, such genetic transformation allows to express a heterologous polypeptide such as a glucoamylase and/or alpha-amylase.

The expression "mutagenesis" designates the process of appearance of a mutation. Classically, two methods are possible: random mutagenesis, and insertional or directed mutagenesis. The first consists of the application of physical treatment (e.g. UV radiation) or treatment by chemical mutagenic agents, which will randomly induce mutations in the genome of the organism studied. The second will use molecular biology methods to produce a specific modification (i.e. promoter, gene, terminator, etc.) either in a region of the genome or on a specific locus. Locus is used to mean a specific and invariable physical location on a chromosome.

The expression "fermentation" or "ferment" refers to a biological process that takes place in an oxygen-deprived, i.e. anaerobic or semi-anaerobic, environment in which the substrate, e.g. glucose, comprised in a biomass, is modified by yeasts. In particular, alcohol (ethanol) fermentation refers to the conversion of the substrate, e.g. glucose, to ethanol. Those skilled in the art will be able to determine the appropriate conditions for alcoholic fermentation. By way of example, reference can be made to the alcoholic fermentation conditions described in the reference book "Yeast Technology", 2nd edition, 1991, G. Reed and T.W. Nagodawithana, published by Van Nostrand Reinhold, ISBN 0-442-31892-8. The fermentation medium comprises the following elements: at least one source of fermentable carbon, at least one source of nitrogen, at least one source of sulfur, at least one source of phosphorus, at least one source of vitamins and/or at least one source of minerals. The carbon source is supplied, for example, in the form of a sugar immediately assimilable by the yeast, such as xylose, arabinose, glucose, fructose or galactose, a sucrose-type disaccharide and/or a mixture of these sugars. These sugars can be supplied in the form of syrup, molasses, EP2 (Poor Drain from 2nd sugar crystallization), hydrolysates of all or part of a plant material and/or a mixture thereof.

The expression "end of fermentation" refers to the stage of fermentation when the economic advantage of continuing fermentation to produce a small amount of additional alcohol is exceeded by the cost of continuing fermentation in terms of fixed and variable costs. In a more general sense, "end of fermentation" refers to the point where a fermentation will no longer produce a significant amount of additional alcohol, i.e., no more than about 1% additional alcohol.

The expression "biomass" refers to all organic material used for fermentation.

As used herein, a "substrate" is a molecule that can be directly or indirectly metabolized to ethanol by fermentation by fermenting organisms, for example *Saccharomyces cerevisiae* strains or any of the yeasts or yeast products described herein. An important feature of the substrate in the frame of the present application is its dry solid content (or gravity) which refers to the proportion of solid material remaining in a sample after all liquid, typically water, has been removed. Said features are usually expressed in weight/weight percentage (w/w%).

The expression "nutrients" are widely used in the art of fermentation and include at least one source of nitrogen, at least one source of sulfur, at least one source of phosphorus, at least one source of vitamins, at least one source of minerals and combinations thereof. The nitrogen source is for example provided in the form of yeast extracts, ammonium sulfate, ammonium hydroxide, di-ammonium phosphate, ammonia, urea, or a combination thereof. The source of phosphorus is for example provided in the form of phosphoric acid, potassium phosphate, di-ammonium phosphate, mono-ammonium phosphate, and/or a combination thereof. The sulfur source is for example provided in the form of ammonium sulfate, magnesium sulfate, sulfuric acid, and/or a combination thereof. The source of vitamins is for example provided in the form of molasses, yeast hydrolysate, a solution of pure vitamins or a mixture of pure vitamins and/or a combination thereof. The source of vitamins supplies the yeast with all vitamins in amounts at least equivalent to those recommended in reference books. Several sources of vitamins can be combined. Such nutrients are generally supplied and in greater proportion than in fermentation.

The expression "batch fermentation" is widely used in the art of fermentation and refers to fermentation in which nutrients and substrate are added at the beginning of the fermentation without adding any more during the fermentation.

The expression "fed-batch fermentation" is widely used in the art of fermentation and refers to fermentation in which nutrients and substrate are added as the organisms multiply, for an extended duration.

The expression "continuous fermentation" is widely used in the art of fermentation and refers to fermentation in which nutrients and substrate are added continuously as per the need and the products are removed as they are formed continuously.

Herein after, the present disclosure is described in further detail and is exemplified.

Described herein, inter alia, are fermenting organisms such as *Saccharomyces* yeast strains for producing a fermentation product, such as ethanol, from starch and/or cellulosic containing material. The Applicants have found new *Saccharomyces cerevisiae* strains having improved ability to produce ethanol, increased temperature tolerance, increased substrate consumption ability, displaying ethanol to glycerol ratio and reduced by-products production to maximize yield. These features are of importance to the industrial production of ethanol from corns.

**Fermenting Organisms**

Fermenting Organisms are organisms able to ferment a substrate such as yeasts, including *Saccharomyces cerevisiae* yeasts. The fermenting organism is selected from the yeast strains described herein and the derivatives thereof.

In one embodiment, the fermenting organism is a *Saccharomyces cerevisiae* strain deposited on December 20, 2024 under the Budapest Treaty at the CNCM (Collection Nationale de Cultures de Microorganismes, Institut Pasteur, 25 rue du Docteur Roux, 75724 Paris Cedex 15) having deposit accession number I-6167 (*Saccharomyces cerevisiae* strain H12), or a derivative thereof.

In another embodiment, the fermenting organism is a Saccharomyces cerevisiae strain deposited on December 20, 2024 under the Budapest Treaty at the CNCM having deposit accession number I-6168 (Saccharomyces cerevisiae strain H147), or a derivative thereof.

In an embodiment, the fermenting organism has one or more defining characteristics and/or fermentation characteristics selected from:

a. a higher ethanol content at the end of fermentation than *Saccharomyces* strain I-4071 under the same fermentation conditions;
b. an increased temperature tolerance than *Saccharomyces* strain I-4071 under the same fermentation conditions;
c. a lower residual substrate percentage, optionally a glucose percentage, at the end of fermentation than *Saccharomyces* strain I-4071 under the same fermentation conditions;
d. a lower glycerol content at the end of fermentation than *Saccharomyces* strain I-4071 under the same fermentation conditions; and
e. a lower glycerol/ethanol ratio than *Saccharomyces* strain I-4071 under the same fermentation conditions.

As used herein, "the same fermentation conditions" refers to conditions of fermentation including the type of substrate, the dry solid content of the substrate, the composition of the growth/fermentation medium, the conditions of the growth/fermentation (temperature, shaking, oxygen supply, pH, nutrients...) and the type of fermentation (fed-batch, batch or continuous).

According to a first embodiment, typical conditions for assessing such characteristics are a temperature of fermentation at 32°C and a dry solid content of the substrate of about 36%, for example of corn mash.

According to a second embodiment, typical conditions for assessing such characteristics are a temperature of the fermentation between 34°C and 39°C and a dry solid content of the substrate of about 33%, for example of corn mash.

In the conditions of the first embodiment, the fermenting organism produces an ethanol content of at least about 18.5% v/v, preferably at least 19% v/v, more preferably et least 19.5% v/v or 20% v/v after 54 hours of fermentation. In a preferred embodiment, the *Saccharomyces cerevisiae* strain number I-6167 (*Saccharomyces cerevisiae* strain H12) or the *Saccharomyces cerevisiae* strain number I-6168 *(Saccharomyces cerevisiae* strain H147), or a derivative thereof produces an ethanol content of at least about 18.5% v/v, preferably at least 19% v/v, more preferably et least 19.5% v/v or 20% v/v after 54 hours of fermentation.

In the conditions of the second embodiment, the fermenting organism produces an ethanol content of at least about 17.5% v/v, preferably at least 18% v/v, more preferably at least 18.2% v/v or 18.3% v/v or 18.4% v/v after 65 hours of fermentation. In a preferred embodiment, the *Saccharomyces cerevisiae* strain number I-6167 (*Saccharomyces cerevisiae* strain H12) or the *Saccharomyces cerevisiae* strain number I-6168 *(Saccharomyces cerevisiae* strain H147), or a derivative thereof produces an ethanol content of at least about 17.5% v/v, preferably at least 18% v/v, more preferably at least 18.2% v/v or 18.4% v/v after 65 hours of fermentation.

Higher ethanol content is the aim of the producers of bioethanol. The amount of ethanol present in the fermentation medium is measured by any appropriate means known to the person skilled in the art. It can be a direct measurement of the ethanol produced or an indirect measurement through a parameter related to ethanol production, such as the loss of mass. For example, the production of alcohol may be measured by chromatography, including HPLC (High Performance Liquid Chromatography), an enzymatic kit (for example the determination of ethanol by Boehringer kit), or a determination by potassium dichromate.

In one embodiment, the fermenting organism displays an increase of the temperature tolerance compared to Saccharomyces cerevisiae strain Ethanol Red® (deposited September 4, 2008 under Accession Number I-4071 at CNCM) under the same process conditions, e.g., conditions described herein.

Increased temperature tolerance is an advantage as the fermentation temperature may fluctuate to some degree. In the early part of fermentation, plants often do not actively heat the fermentation. The temperature may therefore increase naturally from the yeast's metabolism. The plant may use heat exchangers to control early fermentation temperatures, so it does not go too high. During the majority of the year, the plants can easily control the early temperature, and the peak temperature is typically about 32°C. However, during the summer months or in equatorial countries, the cooling water used in heat exchangers is not cold enough to control the temperatures. Therefore, in plants that do not have chillers (i.e. , a water refrigeration system), the early fermentation temperatures can reach above 34°C to 39°C which stresses the yeast. Thus, in a preferred embodiment, the *Saccharomyces cerevisiae* strain number I-6167 (*Saccharomyces cerevisiae* strain H12) and number I-6168 (*Saccharomyces cerevisiae* strain H147), or a derivative thereof increases the temperature tolerance compared to Saccharomyces cerevisiae strain Ethanol Red® (deposited September 4, 2008 under Accession Number I-4071 at CNCM) under the same process conditions, e.g., conditions described herein.

In other words, a fermenting organism as described herein, in particular the *Saccharomyces cerevisiae* strain number I-6167 (*Saccharomyces cerevisiae* strain H12) and number I-6168 (*Saccharomyces cerevisiae* strain H147), or a derivative thereof, at a given temperature, allows to produce more ethanol than *Saccharomyces cerevisiae* strain Ethanol Red® (I-4071) under the same process conditions, e.g., conditions described herein.

In one embodiment, the fermenting organism displays a lower residual substrate percentage, optionally a glucose percentage, at the end of fermentation than Saccharomyces strain I-4071 under the same fermentation conditions.

In an embodiment, the fermenting organism as described herein, in particular the *Saccharomyces cerevisiae* strain number I-6167 (*Saccharomyces cerevisiae* strain H12) or number I-6168 (*Saccharomyces cerevisiae* strain H147), or a derivative thereof, displays a reduced residual glucose percentage at the end of fermentation compared to *Saccharomyces cerevisiae* strain Ethanol Red® (deposited September 4, 2008 under Accession Number I-4071 at CNCM) under the same process conditions, e.g., conditions described herein.

In the conditions of the first embodiment, the fermenting organism displays a residual glucose percentage after 54 hours of fermentation of at most 2% w/v, preferably at most 1.5% w/v, more preferably at most 1% w/v, even more preferably at most 0.5% w/v or at most 0.4% w/v or at most 0.3% w/v or at most 0.2% w/v. In a preferred embodiment, the *Saccharomyces cerevisiae* strain number I-6167 (*Saccharomyces cerevisiae* strain H12) or the *Saccharomyces cerevisiae* strain number I-6168 *(Saccharomyces cerevisiae* strain H147), or a derivative thereof displays a residual glucose percentage after 54 hours of fermentation of at most 2% w/v, preferably at most 1.5% w/v, more preferably at most 1% v/v, even more preferably at most 0.5% w/v or at most 0.4% w/v or at most 0.3% w/v or at most 0.2% w/v.

In the conditions of the second embodiment, the fermenting organism displays a residual glucose percentage after 65 hours of fermentation of at most 1.9% w/v, preferably at most 1.5% w/v, more preferably at most 1% w/v, even more preferably at most 0.5% w/v or at most 0.4% w/v or at most 0.3% w/v or at most 0.2% w/v. In a preferred embodiment, the *Saccharomyces cerevisiae* strain number I-6167 *(Saccharomyces cerevisiae* strain H12) or the *Saccharomyces cerevisiae* strain number I-6168 (*Saccharomyces cerevisiae* strain H147), or a derivative thereof displays a residual glucose percentage after 65 hours of fermentation of at most 1.9% w/v, preferably at most 1.5% w/v, more preferably at most 1% w/v, even more preferably at most 0.5% w/v or at most 0.4% w/v or at most 0.3% w/v or at most 0.2% w/v.

Reducing the residual substrate percentage, i.e. glucose, at the end of fermentation means that the substrate, i.e. glucose, is converted to ethanol as much as possible. It is preferred that the fermenting organism converts all of the substrates of the medium to ethanol, and that the overall yield of conversion of the consumed substrate to ethanol is as high as possible and, consequently, the fewest coproducts such as glycerol are generated during the fermentation.

In one embodiment, the fermenting organism displays a similar or lower glycerol content at the end of fermentation than Saccharomyces strain I-4071 under the same fermentation conditions.

In the conditions of the first embodiment, the fermenting organism displays a glycerol content after 54 hours of fermentation of at most 16 g/L, preferably at most 15.5 g/L, more preferably at most 15.2 g/L. In a preferred embodiment, the *Saccharomyces cerevisiae* strain number I-6167 (*Saccharomyces cerevisiae* strain H12) or the *Saccharomyces cerevisiae* strain number I-6168 (*Saccharomyces cerevisiae* strain H147), or a derivative thereof displays a glycerol content after 54 hours of fermentation of at most 16 g/L, preferably at most 15.5 g/L, more preferably at most 15.2 g/L. In another embodiment, the *Saccharomyces cerevisiae* strain number I-6168 (*Saccharomyces cerevisiae* strain H147), or a derivative thereof displays a glycerol content after 54 hours of fermentation of at most 16 g/L, preferably at most 15.5 g/L, more preferably at most 15 g/L, even more preferably at most 14 g/L or 13 g/L or 12.8 g/L.

In the conditions of the second embodiment, the fermenting organism displays a glycerol content after 65 hours of fermentation of at most 11 g/L, preferably at most 10.5 g/L, more preferably at most 10.4 g/L, even more preferably at most 10.3 g/L. In a preferred embodiment, the *Saccharomyces cerevisiae* strain number I-6167 (*Saccharomyces cerevisiae* strain H12) or the *Saccharomyces cerevisiae* strain number I-6168 (*Saccharomyces cerevisiae* strain H147), or a derivative thereof displays a glycerol content after 65 hours of fermentation of at most 11 g/L, preferably at most 10.5 g/L, more preferably at most 10.4 g/L, even more preferably at most 10.3 g/L. In another embodiment, the *Saccharomyces cerevisiae* strain number I-6168 (*Saccharomyces cerevisiae* strain H147), or a derivative thereof displays a glycerol content after 65 hours of fermentation of at most 11 g/L, preferably at most 10.5 g/L, more preferably at most 10 g/L, even more preferably at most 9.5 g/L or 9.3 g/L or 9.15g/L or 9.11 g/L.

In one embodiment, the fermenting organism displays a similar or lower glycerol/ethanol ratio than *Sacchar-*

*omyces* strain I-4071 under the same fermentation conditions.

In the conditions of the first embodiment, the fermenting organism displays a glycerol/ethanol ratio after 54 hours of fermentation of at most 0.1 g/g, preferably at most 0.098 g/g, more preferably at most 0.097 g/g. In a preferred embodiment, the *Saccharomyces cerevisiae* strain number I-6167 (*Saccharomyces cerevisiae* strain H12) or the *Saccharomyces cerevisiae* strain number I-6168 (*Saccharomyces cerevisiae* strain H147), or a derivative thereof displays a glycerol/ethanol ratio after 54 hours of fermentation of at most 0.1 g/g, preferably at most 0.098 g/g, more preferably at most 0.097 g/g. In another embodiment, the *Saccharomyces cerevisiae* strain number I-6168 (*Saccharomyces cerevisiae* strain H147), or a derivative thereof displays a glycerol/ethanol ratio after 54 hours of fermentation of at most 0.1 g/g, preferably at most 0.098 g/g, more preferably at most 0.097 g/g, even more preferably at most 0.090 g/g, or 0.085 g/g, or 0.080 g/g.

In the conditions of the second embodiment, the fermenting organism displays a glycerol/ethanol ratio after 65 hours of fermentation of at most 0.075 g/g, preferably at most 0.072 g/g, more preferably at most 0.071 g/g. In a preferred embodiment, the *Saccharomyces cerevisiae* strain number I-6167 (*Saccharomyces cerevisiae* strain H12) or the *Saccharomyces cerevisiae* strain number I-6168 (*Saccharomyces cerevisiae* strain H147), or a derivative thereof displays a glycerol/ethanol ratio after 65 hours of fermentation of at most 0.075 g/g, preferably at most 0.072 g/g, more preferably at most 0.071 g/g. In another embodiment, the *Saccharomyces cerevisiae* strain number I-6168 (*Saccharomyces cerevisiae* strain H147), or a derivative thereof displays a glycerol/ethanol ratio after 65 hours of fermentation of at most 0.075 g/g, preferably at most 0.072 g/g, more preferably at most 0.070 g/g, even more preferably at most 0.065 g/g, or 0.064 g/g or 0.063 g/g or 0.062 g/g.

*Saccharomyces cerevisiae* yeast strains

These yeast strains are referred to herein as "the yeast strains", "the *Saccharomyces* yeast strains", *"Saccharomyces cerevisiae* strains" or by their designations (i.e. "H12" or "H147"). The yeast strains (i.e. "H12" or "H147") were produced from one or more different *Saccharomyces* yeast strains by one or more of the methods as shown in another application WO2015/121595.

In one aspect, the yeast strains described herein comprise one or more defining characteristics and/or fermentation characteristics including a higher ethanol content at the end of fermentation, an increased temperature tolerance, a lower residual substrate percentage, a lower glycerol content at the end of fermentation, and a lower ratio glycerol/ethanol, than other yeast strains and typical yeast strains used for fermentation, in particular in comparison to the yeast strain deposited September 4, 2008 under Accession Number I-4071 at CNCM, the yeast strain used in the product Ethanol Red®. Representative samples of the yeast strains have been deposited on December 20, 2024 under the above-identified accession numbers I-6168 and I-6167 at the CNCM (Collection Nationale de Cultures de Microorganismes, Institut Pasteur, 25 rue du Docteur Roux, 75724 Paris Cedex 15).

*Saccharomyces cerevisiae* yeast strains derivatives

The fermenting organism may also be a derivative of *Saccharomyces cerevisiae* strain H12 (deposited under CNCM Number I-6167) or H147 (deposited under CNCM Number I-6168).

*Crossing derivatives*

In one embodiment, a derivative of *Saccharomyces cerevisiae* strain H12, or H147 is derived by crossing *Saccharomyces cerevisiae* strain H12 with *Saccharomyces cerevisiae* strain H147, or *Saccharomyces cerevisiae* strain H12 with *Saccharomyces cerevisiae* strain H12, or *Saccharomyces cerevisiae* strain H147 with *Saccharomyces cerevisiae* strain H147, or *Saccharomyces cerevisiae* strain H12 or H147 with another yeast strain not according to the invention. For example, the derivate is a hybrid strain produced by culturing a first yeast strain with *Saccharomyces cerevisiae* strain H12, or H147 under conditions which permit combining of DNA between the first yeast strain and *Saccharomyces cerevisiae* strain H12, or H147.

In one embodiment, such derivative may be produced by:

(a) culturing a first yeast strain with a second yeast strain, wherein the second yeast strain is *Saccharomyces cerevisiae* strain H12 (or a derivative of *Saccharomyces cerevisiae* strain H12) under conditions which permit combining of DNA between the first yeast strain and the second yeast strain;

(b) screening or selecting for a derivative of *Saccharomyces cerevisiae* strain H12, such as screening or selecting for a derivative with increased ethanol production in corn mash compared to the first strain under the same fermentation conditions, for example as described herein;

(c) optionally repeating steps (a) and (b) with the screened or selected strain as the first yeast strain and/or the second yeast strain, until a derivative of *Saccharomyces cerevisiae* strain H12 is obtained which exhibits one or more defining characteristics and/or fermentation characteristics of *Saccharomyces cerevisiae* strain H12.

In another embodiment, such derivative may be produced by:

(a) culturing a first yeast strain with a second yeast strain, wherein the second yeast strain is *Saccharomyces cerevisiae* strain H147 (or a derivative of *Saccharomyces cerevisiae* strain H147) under conditions which permit combining of DNA between the first yeast strain and the second yeast strain;

(b) screening or selecting for a derivative of *Saccharomyces cerevisiae* strain H147, such as screening or selecting for a derivative with increased ethanol production in corn mash compared to the first strain, under the same fermentation conditions, for example as described herein;

(c) optionally repeating steps (a) and (b) with the screened or selected strain as the first yeast strain and/or the second yeast strain, until a derivative of *Saccharomyces cerevisiae* strain H147 is obtained which exhibits one or more defining characteristics and/or fermentation characteristics of *Saccharomyces cerevisiae* strain H147.

Typically, the first yeast strain is a *Saccharomyces* strain. More typically, the first yeast strain is a *Saccharomyces cerevisiae* strain. The first yeast strain may have desired properties which are sought to be combined with the defining characteristics and/or fermentation characteristics of *Saccharomyces cerevisiae* strain H12, or H147. The first yeast strain may be, for example, any *Saccharomyces cerevisiae* strain, such as for example Ethanol Red®. It will also be appreciated that the first yeast strain may be *Saccharomyces cerevisiae* strain H12, or H147 (or a derivative of *Saccharomyces cerevisiae* strain H12, or H147).

The first and second yeast strains are cultured under conditions which permit combining of DNA between the yeast strains. As used herein, "combining of DNA" between yeast strains refers to combining of all or a part of the genome of the yeast strains. Combining of DNA between yeast strains may be by any method suitable for combining DNA of at least two yeast cells, and may include, for example, mating methods which comprise sporulation of the yeast strains to produce haploid cells and subsequent hybridising of compatible haploid cells; cytoduction; or cell fusion such as protoplast fusion.

In one embodiment, culturing the first yeast strain with the second yeast, under conditions which permit combining of DNA between the first yeast strain and the second yeast strain, comprises:

(i) sporulating the first yeast strain and the second yeast strain;

(ii) germinating and hybridizing spores produced by the first yeast strain with spores produced by the second yeast strain.

In another embodiment, such derivative may be produced by:

(a) providing: (i) a first yeast strain; and (ii) a second yeast strain, wherein the second yeast strain is *Saccharomyces cerevisiae* strain H12 or H147 (or a derivative of *Saccharomyces cerevisiae* strain H12 or H147);

(b) sporulating the first yeast strain and the second yeast strain;

(c) germinating and hybridizing the spores of the first yeast strain with germinated spores of the second yeast strain;

(d) screening or selecting for a derivative of *Saccharomyces cerevisiae* strain H12 or H147, such as screening or selecting for a derivative with increased ethanol production compared to the first strain, and/or higher ethanol yield from glucose during fermentation of corn mash than the first strain, under the same fermentation conditions, for example as described herein;

(e) optionally repeating steps (b) to (d) with the screened or selected strain as the first and/or second yeast strain.

Methods for sporulating, germinating and hybridizing yeast strains, and in particular, *Saccharomyces* strains, are known in the art and are described in, for example, Ausubel, F. M. et al., (1997) Current Protocols in Molecular Biology, Volume 2, pages 13.2.1 to 13.2.5 (John Willey & Sons Inc); Chapter 7, "Sporulation and Hybridisation of yeast" by R.R. Fowell, in "The Yeasts" vol 1 , A.H. Rose and J.S. Harrison (Eds), 1969, Academic Press.

In one embodiment, the yeast strains may be cultured under conditions which permit cell fusion. Methods for the generation of intraspecific or interspecific hybrids using cell fusion techniques are described in, for example, Spencer et al. (1990) in, Yeast Technology, Spencer JFT and Spencer DM (Eds), Springer Verlag, New York.

In another embodiment, the yeast strains may be cultured under conditions which permit cytoduction. Methods for cytoduction are described in, for example, Inge-Vechymov et al. (1986) Genetika 22: 2625-2636; Johnston (1990) in, Yeast technology, Spencer JFT and Spencer DM (Eds), Springer Verlag, New York.

In one embodiment, screening or selecting for derivatives of *Saccharomyces cerevisiae* strain H12, or H147 comprises screening or selecting for a derivative with increased ethanol production compared to the first strain, and/or screening or selecting for a hybrid which has a higher ethanol yield, e.g., as described in WO2015/101753.

*Mutation derivatives*

In one embodiment, a derivative of *Saccharomyces cerevisiae* strain H12, or H147 is derived by mutation, said mutation being spontaneous in the genome thereof or induced by mutagenesis to provide a mutant.

In one embodiment, a derivative of *Saccharomyces cerevisiae* strain H12, or H147, may be a mutant of *Saccharomyces cerevisiae* strain H12, or H147 wherein the mutant yeast is mutated comprising contacting the yeast strain with a mutagen. In one embodiment, the mutagen is ethyl methanesulfonate (EMS), ultraviolet light (UV), X-rays, methylmethane sulphonate (MMS), nitrous acid, nitrosoguanidine (NNG), acridine mustard, 2-methoxy-6-chloro-9 [3-(ethyl-2-chloroethyl)aminopropylamino]acridine-2 (ICR-170), or nitrogen mustard.

Methods for producing mutants of *Saccharomyces* yeast, and specifically mutants of *Saccharomyces cerevisiae,* are known in the art and described in, for example, Lawrence C.W. (1991) Methods in Enzymology, 194: 273-281.

*Genetic transformation derivatives*

In one embodiment, a derivative of *Saccharomyces cerevisiae* strain H12, or H147 is derived by genetic transformation to provide a recombinant yeast. In one embodiment, the recombinant yeast comprises a modification to suppress expression of a gene, enhance expression of a gene, introduce a gene, or delete a gene.

In another embodiment, a derivative of *Saccharomyces cerevisiae* strain H12, or H147 may be a recombinant derivative of *Saccharomyces cerevisiae* strain H12, or H147.

A recombinant derivative of *Saccharomyces cerevisiae* strain H12, or H147 is a strain produced by introducing into *Saccharomyces cerevisiae* strain H12, or H147 a nucleic acid using recombinant DNA technology. Recombinant methods for the introduction of nucleic acid into *Saccharomyces* yeast cells, and in particular strains of *Saccharomyces,* are known in the art and are described in, for example, Ausubel, F. M. et al. (1997), Current Protocols in Molecular Biology, Volume 2, pages 13.7.1 to 13.7.7, published by John Wiley & Sons Inc.

In one embodiment, a recombinant derivative of *Saccharomyces cerevisiae* strain H12, or H147 can be prepared by genetically modifying the strain (or another derivative thereof) to express a heterologous enzyme, such as an alphaamylase and/or glucoamylase described herein (or any enzyme described in WO2017037362 and WO2020007823, the content of which is incorporated herein by reference).

In another embodiment, a recombinant derivative of *Saccharomyces cerevisiae* strain H12, or H147 has been prepared by genetically modifying the strain (or another derivative thereof) to express a heterologous enzyme such as xylose isomerase (XI) and/or xylose reductase (XR or XYL1) and/or xylitol dehydrogenase (XDH or XYL2) and/or D-xylulokinase as described in another application WO2015/121595 or WO2016/174349 the content of which is incorporated herein by reference. For example, *Saccharomyces cerevisiae,* which is a yeast commonly used by producers of bioethanol, is not capable of metabolizing D-xylose but can absorb it via non-specific carriers. It is transported by hexose transporters that have a lower affinity for D-xylose than for D-glucose. Thus, it can be attractive not only to genetically modify the cell to give it the capacity to metabolize D-xylose, but also to transform it so that it absorbs said sugar more effectively.

In another embodiment, a recombinant derivative of *Saccharomyces cerevisiae* strain H12, or H147 has been prepared by genetically modifying the strain (or another derivative thereof) to express a heterologous enzyme such as arabinose isomerase and/or ribulokinase and/or a ribulose 5 phosphate epimerase as described in another application WO2018/138135 the content of which is incorporated herein by reference. For example, *Saccharomyces cerevisiae,* which is a yeast commonly used by producers of bioethanol, is not capable of metabolizing arabinose naturally or at a very low level, not industrially applicable. Indeed, L-arabinose is converted into L-ribulose under the action of an arabinose isomerase (araA: EC: 5.3.1.4). L-ribulose can itself be converted into L-ribulose-5-phosphate under the action of a ribulokinase (araB; EC: 2.7.1.16). L-ribulose-5-phosphate can thus be transformed into D-xylulose-5-phosphate by a ribulose 5 phosphate epimerase (araD; EC: 5.1.3.4). Advantageously, D-xylulose-5-phosphate is carried out by the non-oxydative part of the pentose phosphates pathway and can result in production of ethanol. Thus, it can be attractive not only to genetically modify the cell to give it the capacity to metabolize arabinose but also to metabolize L-ribulose and/or L-ribulose-5-phosphate and/or into D-xylulose-5-phosphate.

Indeed, xylose and arabinose are sugars obtained from lignocellulose coming from agricultural and agro-industrial activity. Lignocellulose is a complex substrate made of three main fractions which are cellulose, hemicellulose and lignin. This concerns recyclable waste used in the manufacture of ethanol, the demand for which continues to rise due, for example, to its use as a biofuel. Therefore, producing ethanol from lignocellulose biomass is of economic and environmental importance, as it enables waste to be recycled.

In one embodiment, is a method of producing a recombinant derivative of *Saccharomyces cerevisiae* strain H12 (deposited under Accession Number CNCM I-6167) comprising:

(a) transforming *Saccharomyces cerevisiae* strain H12 (or a derivative of *Saccharomyces cerevisiae* strain H12) with one or more expression vectors encoding a heterologous enzymes, as mentioned above, and

(b) isolating the transformed strain.

In one embodiment, a derivative of *Saccharomyces cerevisiae* strain H12 may be prepared by:

(a) culturing a first yeast strain with a second yeast strain, wherein the second yeast strain is *Saccharomyces cerevisiae* strain H12 (or a derivative of *Saccharomyces cerevisiae* strain H12), under conditions which permit combining of DNA between the first yeast strain and the second yeast strain; and

(b) isolating hybrid strains; and

(c) optionally repeating steps (a) and (b) using a hybrid strain isolated in step (b) as the first yeast strain and/or the derivative of *Saccharomyces cerevisiae* strain H12.

In one embodiment, is a method of producing a recombinant derivative of *Saccharomyces cerevisiae* strain H147 (deposited under Accession Number CNCM I-6168) comprising:

(a) transforming *Saccharomyces cerevisiae* strain H147 (or a derivative of *Saccharomyces cerevisiae* strain H1247 with one or more expression vectors encoding a heterologous enzymes, as mentioned above; and

(b) isolating the transformed strain.

In one embodiment, a derivative of *Saccharomyces cerevisiae* strain H147 may be prepared by:

(a) culturing a first yeast strain with a second yeast strain, wherein the second yeast strain is *Saccharomyces cerevisiae* strain H147 (or a derivative of *Saccharomyces cerevisiae* strain H147), under conditions which permit combining of DNA between the first yeast strain and the second yeast strain; and

(b) isolating hybrid strains; and

(c) optionally repeating steps (a) and (b) using a hybrid strain isolated in step (b) as the first yeast strain and/or the derivative of *Saccharomyces cerevisiae* strain H147.

In some embodiments, the derivative of *Saccharomyces cerevisiae* strain H12, or H147, expresses a glucoamylase and/or an alpha-amylase. The derivatives expressing heterologous enzymes as mentioned above have been generated in order to improve ethanol yield and to improve process economy by cutting enzyme costs since part or all of the necessary enzymes needed to hydrolyse starch will be produced by the yeast organism.

In an embodiment, the fermenting organism as described herein is a recombinant produced by genetically modifying the fermenting organism as described herein to express a heterologous enzyme from the group consisting of alphaamylase, glucoamylase xylose isomerase, xylose reductase, xylitol dehydrogenase, D-xylulokinase, arabinose isomerase, ribulokinase, a ribulose 5 phosphate epimerase, and combinations thereof.

## Compositions

This aspect relates to a composition comprising the fermenting organism described herein and naturally occurring and/or none naturally occurring components. In one embodiment, the composition comprises the fermenting organism as described herein and one or more components selected from the group consisting of surfactants, emulsifiers, gums, swelling agents, protectants and antioxidants and other processing aids. In a preferred embodiment, is a composition comprising a *Saccharomyces* yeast described herein, in particular *Saccharomyces cerevisiae* strain H12 or H147, and one or more of the components selected from the group consisting of surfactants, emulsifiers, gums, swelling agents, protectants and antioxidants and other processing aids.

In another embodiment, is a composition comprising *Saccharomyces cerevisiae* strain H12 or H147 (or a derivative of *Saccharomyces cerevisiae* strain H12 or H147). The composition may be, for example, cream yeast, compressed yeast, wet yeast, dry yeast, semi-dried yeast, crumble yeast, stabilized liquid yeast or frozen yeast. Methods for preparing such yeast compositions are known in the art.

In one embodiment, the *Saccharomyces cerevisiae* strain H12 or H147 (or a derivative of *Saccharomyces cerevisiae* strain H12 or H147) is in the form of dry yeast, such as active dry yeast or instant yeast, or in the form of liquid yeast or in the form of pressed yeast. In a preferred embodiment, the *Saccharomyces cerevisiae* strain H12 or H147 (or a derivative of *Saccharomyces cerevisiae* strain H12 or H147) is in the form of dry yeast, and preferably in the form of instant dry yeast.

*Surfactant*

The compositions described herein may comprise the fermenting organism described herein, in particular *Saccharomyces cerevisiae* strain H12, or H147, and any suitable surfactants. In one embodiment, the surfactant(s) is/are an anionic surfactant, cationic surfactant, and/or nonionic surfactant.

*Emulsifier*

The compositions described herein may comprise the fermenting organism described herein, in particular *Saccharomyces cerevisiae* strain H12, or H147, and any suitable emulsifier. In one embodiment, the emulsifier is a fatty-acid ester of sorbitan. In one embodiment, the emulsifier is selected from the group of sorbitan monostearate (SMS), citric acid esters of monodiglycerides, polyglycerolester, fatty acid esters of propylene glycol.

In one embodiment, the composition comprises the fermenting organism described herein, in particular *Saccharomyces cerevisiae* strain H12, or H147, and Olindronal SMS, Olindronal SK, or Olindronal SPL including composition concerned in European Patent no. 1 ,724,336 (hereby incorporated by reference). These products are commercially available from Bussetti, Austria, for active dry yeast.

*Gum*

The compositions described herein may comprise the fermenting organism described herein, in particular *Saccharomyces cerevisiae* strain H12, or H147, and any suitable gum. In one embodiment, the gum is selected from the group of carob, guar, tragacanth, arabic, xanthan and acacia gum, in particular for cream, compressed and dry yeast.

*Swelling Agents*

The compositions described herein may comprise the fermenting organism described herein, in particular *Saccharomyces cerevisiae* strain H12, or H147, and any suitable swelling agent. In one embodiment, the swelling agent is methyl cellulose or carboxymethyl cellulose.

*Protectants*

The compositions described herein may comprise the fermenting organism described herein, in particular *Saccharomyces cerevisiae* strain H12, or H147, and any suitable protectant. In an embodiment the protectant is glycerol.

*Antioxidant*

The compositions described herein may comprise the fermenting organism described herein, in particular *Saccharomyces cerevisiae* strain H12, or H147, and any suitable antioxidant. In one embodiment, the antioxidant is butylated hydroxyanisol (BHA) and/or butylated hydroxytoluene (BHT), or ascorbic acid (vitamin C), particular for active dry yeast.

In one aspect, the composition comprises one or more defining characteristics and/or fermentation characteristics including a higher ethanol content at the end of fermentation, an increased temperature tolerance, a lower residual substrate percentage, a lower glycerol content at the end of fermentation, a lower ratio glycerol/ethanol, than other yeast strains and typical yeast strains used for fermentation, in particular in comparison to composition comprising the yeast strain I-4071 used in the product Ethanol Red® as described above.

In addition, the compositions described herein can be readily distinguished from other yeast products used in the ethanol industry that do not have the ethanol producing capabilities and defining characteristics and/or fermentation characteristics of the compositions described herein.

**Process for producing ethanol from a biomass**

Another aspect of the disclosure provides a process for producing ethanol from a biomass comprising contacting the biomass with the fermenting organism or composition as described herein.

In one embodiment, the ethanol is used for fuel ethanol, industrial ethanol, potable ethanol, or a combination thereof.

Fuel ethanol is manufactured for use in internal combustion engines and may manufactured as anhydrous or hydrous fuel ethanol. Anhydrous fuel ethanol can be mixed with gasoline to form an ethanol/gasoline mixture or with diesel to form an ethanol/diesel mixture. Hydrous fuel ethanol can be used directly as a fuel in internal combustion engines.

Industrial ethanol is manufactured for use in a variety of applications including as a solvent in pharmaceuticals, cosmetics, detergents, household cleaners and disinfectants, and coatings and inks; and as a chemical intermediate in manufacture of ethyl acetate, ethyl acrylate, polyethylene, acetic acid, and other organic molecules of industrial importance.

Potable ethanol is manufactured for human consumption and includes the ethanol found in wine, beer, cider, sake, mead, kombucha, and distilled spirits including whisky, bourbon, cachaça, Chinese white liquor, baijiu, and others

Classical conditions of the process for producing ethanol may comprise a step of fermentation in anaerobic or semi-anaerobic conditions by a fermenting organism or composition as described herein in a fermentation medium. The fermentation being an alcoholic fermentation. The person skilled in art knows how to determine the appropriate conditions for alcoholic fermentation.

For example, one can refer to the alcoholic fermentation conditions described in the reference book "Yeast Technology", 2nd edition, 1991, G. Reed and T. W. Nagodawithana, published by Van Nostrand Reinhold, ISBN 0-442-31892-8.

The fermentation medium includes a fermentation substrate, that is, the carbohydrate source that is metabolized by the fermenting organism as described herein.

In one embodiment, the fermenting organism or derivative thereof, or composition as described herein is incubated with a substrate comprising fermentable sugars from a biomass such as a plant biomass from forests and/or from agricultural or food-processing products and/or coproducts that constitute a considerable source of carbon for the production of molecules of interest. The fermenting organism or derivative thereof is incubated with the substrate under conditions that allow fermentation of the fermentable sugars.

The substrate is a carbohydrate source which is fermentable sugar, starch or cellulose. Sugars may be with 6 carbon atoms, also called C6 sugars, or with 5 carbon atoms, also called C5 sugars. C6 sugars comprises glucose, galactose, maltose, fructose, sucrose, mannose, or a combination thereof, typically glucose. C5 sugars comprises ribulose, xylulose, arabinose, xylose, or a combination thereof, typically xylose and arabinose. In a preferred embodiment, the substrate is glucose.

The source of the substrate may be any source which contains said fermentable sugar also called sugar containing raw material, for example, from any one or more of the following sources: grain mash material, starch material, cellulose material or other fermentable sugar material.

Grain mash may be corn mash or synthetic corn medium. Starch material, cellulose material or other fermentable sugar material may be selected from the group consisting of hydrolyzed starch, hydrolyzed cellulose, molasses (from sugar cane or sugar beet), sugar cane juice, agave, sugar beet juice, grape juice, fruit juice, hydrolyzed maltodextrins, raw sugar juice, or any other forms of fermentable sugar, starch or cellulose, or combinations thereof. Starch may be obtained from any starch rich crops. Examples of starch rich crops include, but are not limited to, corn, wheat, barley, cassava, sorghum, sweet potato, millet, rice, or any other starch rich crops. In preparing the substrate, the crop may be typically crushed and mixed with water and hydrolytic enzyme(s) under conditions which result in hydrolysis of the starch and release of fermentable sugars such as glucose. Typical enzymes for hydrolysis of the starch include $\alpha$-amylase, amyloglucosidase, pullulanase, $\beta$-amylase, glucoamylase, or mixtures thereof.

In a preferred embodiment, the substrate is provided in the form of corn mash or a Synthetic Corn Medium (SCM). Methods for preparation of corn mash are known in the art and are described in, for example, Thomas et al., (2001) Journal of Applied Microbiology, 90, 819-828. Methods for preparation of substrates similar in function to SCM are known in the art and are described in, for example, U.S. Pat. Number 10,106,823, which is incorporated herein by reference in its entirety. Methods for the preparation of starch-based substrates are also described in, for example, PCT Publication Number 2006/113683 or U.S. Patent Publication Number 2007/0014905.

Generally, fermenting organisms such as yeast, including *Saccharomyces cerevisiae* yeast, require an adequate nutrient for propagation and fermentation. Therefore, in an embodiment, the fermentation medium also comprises nutrients for the fermenting organism. Nutrients are widely used in the art of fermentation and include at least one source of nitrogen, at least one source of sulfur, at least one source of phosphorus, at least one source of vitamins, at least one source of minerals and combinations thereof.

The nitrogen source is for example organic, such as urea or corn mash or yeast extracts, or inorganic, such as ammonia or ammonium hydroxide or ammonium salts such as ammonium sulfate, ammonium hydroxide, di-ammonium phosphate, or a combination thereof.

The source of phosphorus is for example provided in the form of phosphoric acid, potassium phosphate, di-ammonium phosphate, mono-ammonium phosphate, and/or a combination thereof.

The sulfur source is for example provided in the form of ammonium sulfate, magnesium sulfate, sulfuric acid, and/or a combination thereof.

The source of vitamins is for example provided in the form of corn steep liquor, molasses, yeast hydrolysate, a solution of pure vitamins or a mixture of pure vitamins and/or a combination thereof. The source of vitamins supplies the yeast with all vitamins in amounts at least equivalent to those recommended in reference books. Several sources of vitamins can be combined. Such nutrients are generally supplied and in greater proportion than in fermentation.

The mineral source is supplied, for example, in the form of molasses, a mixture of mineral salts and/or their combination. The mineral source provides the yeast with all the macro and trace elements in quantities at least equivalent to those recommended in the reference works. Several mineral sources may be combined. The same substance can provide several different elements.

The substrate content of the fermentation medium may be adjusted so that it is as high as possible i.e. high solid content or high gravity, while at the same time ensuring that the substrate is converted to ethanol as rapidly and as completely as possible. It is preferred that the fermenting organism or composition as described herein convert all of the substrate of the medium to ethanol, and that the overall yield of conversion of the consumed substrate to ethanol is as high as possible and, consequently, the fewest coproducts such as glycerol are generated during the fermentation. In addition, having the highest possible solid content or gravity allows to reduce steam consumption for the distillation stage, which in turn leads to lower energy consumption during distillation, which is both economically and environmentally beneficial.

In a preferred embodiment, the fermentation is conducted with high dry solid content or high gravity or high dry matter of at least 30%, preferably between 32% and 38%, more preferably between 33% and 36%. Said dry solid content may be measured using a technique well known in the field. A measurement protocol is illustrated in the examples.

The fermentation is carried out at a temperature which permits fermentation of the fermentable substrate. In general, the higher the temperature at which the fermentation can be carried out, the more economical the industrial process. Typically, the temperature at which the fermentation is carried out is from 32-39° C. Suitable temperature ranges include 33-39° C., 32-38° C., 33-38° C., 32-37° C., 33-37° C., 32-36° C., 33-36° C., 32-35° C., or 33-35° C.

In a preferred embodiment, the temperature of the fermentation is 32°C or between 34°C and 39°C.

In an embodiment, when the temperature of the fermentation is 32°C, the dry solid content or gravity is 36%.

In another embodiment, when the temperature of the fermentation is between 34°C to 39°C, the solid content or gravity is 33%.

In one embodiment, the end of fermentation is the stage of fermentation when the economic advantage of continuing fermentation to produce a small amount of additional alcohol is exceeded by the cost of continuing fermentation in terms of fixed and variable costs. Thus, the moment where fermentation will no longer produce a significant amount of additional alcohol, i.e., no more than about 1% additional alcohol. For example, the end of fermentation may be from 50 to 70 hours. In a preferred embodiment, the end of fermentation is between 52 and 68 hours, preferably between 54 and 56 hours. In an embodiment, the end of fermentation is 54 hours when the temperature is 32°C and the solid content or gravity is 36%. In another embodiment, the end of fermentation is 65h when the temperature is between 34°C to 39°C and the solid content or gravity is 33%.

Methods for fermentation and distillation are known in the art and are described in, for example, WO 2006/113683 or US 2007/0014905.

In particular, ethanol production as described herein may be carried out using simultaneous saccharification and fermentation ("SSF"), batch fermentation, fed-batch or continuous fermentation.

## FIGURES

**Figure 1:** Results at the end of batch fermentation regarding ethanol content, ratio glycerol/ethanol and residual glucose percentage - corn mash solid content 36% / 32°C.

**Figure 2:** Results at the end of batch fermentation regarding ethanol content, ratio glycerol/ethanol and residual glucose percentage - corn mash solid content 33% / 34-39°C.

**Figure 3:** Results at the end of fed-batch and batch fermentations regarding ethanol content, ratio glycerol/ethanol and residual glucose percentage - corn mash solid content 36% / 32°C.

**EXAMPLES**

Hereinafter, the present invention is described in more details and specifically with reference to the examples, which however are not intended to limit the present invention.

**Example 1:**

Two tests have been carried out to confirm ethanol production capacity of the yeast strains of the present invention in comparison to Ethanol Red® deposited under Accession Number I-4071 at CNCM.

On one hand, the strains were tested in temperate zone conditions, at 32°C and 36% dry solid content which makes it possible to represent the use of strains in temperate zone countries.

On the other hand, the strains were tested in equatorial zone conditions, between 34-39°C and 33% dry solid content which makes it possible to represent the use of strains in equatorial zone countries.

*Materials and methods*

Strains:

   Yeast : *Saccharomyces cerevisiae*
   Strains: I-6168 (*Saccharomyces cerevisiae* strain H147) or I-6167 (*Saccharomyces cerevisiae* strain H12)

Yeast strains have been put into contact with 10g/L of Yeast extract, 20g/L of Peptone (YP), 20g/L of glucose, 20 g/L agar, and incubated at 30°C for 72h.

Then, a single colony has been inoculated in a 250mL flask with 50 ml YP 80g/L glucose and incubated 22 hours at 32°C and shacked at 200 rpm.

Measure of the dry solid content or gravity:

The measure of dry solid content is conducted in corn mash prior to fermentation.

*Apparatus and lab supplies*

   1.1 A laboratory oven
   1.2 Aluminum weigh cans
   1.3 Desiccator
   1.4 Weighing balance with accuracy of 3 decimal places

*Method*

   1.1. Corn mash has been thawed to room temperature and the temperature of oven has been set to 105°C.
   1.2. The weight of aluminum can (A) has been measured in a weighing balance with closed doors.
   1.3. Around 2 g mash sample has been added and the mash has been spread in the weighing can to avoid clumping in a localized area.
   1.4. The weight of can with sample (B) has been measured.
   1.5. The samples have been put in 105°C oven for 3 hours. (Transfer of all cans in a desiccator from weighing balance to the oven).
   1.6. After 3 hours, the cans have been taken out and placed in desiccator to let them cool down to room temperature.
   1.7. The weight of can with dried sample (C) has been measured.
   1.8. Moisture and solids content have been calculated according to the following:

$$Moisture\ content\ (\%) = \left[\frac{B-C}{B-A}\right] * 100$$

$$Solid\ content\ (\%) = 100 - Moisture\ content\ (\%)$$

Mash preparation:

Corn mash (325g/L initial sugar) has been thawed for 3h at 105°C in water bath and supplemented under stirring with:

- 300 ppm N from Urea
- 3 ppm Zn from $ZnSO_4.7H_2O$
- 9 ppm Mg from $MgSO_4.7H_2O$
- Fill flasks with 110g mash

Preparation of yeast suspension for pitching:

Grown cultures have been spanned down 3 min at 3000 rpm, and the supernatant has been discarded. The yeast pellet has been resuspended with ± 5 ml Saline water (0.9% NaCl). yeast suspension has been diluted several time and counted.

Start fermentation:

Yeast suspension and 100 μl of Gluco-amylase (18% v/v) has been added to the mash in the flasks until required pitching rate. Water has been added to get to the correct mash dilution (depending on conditions). The flask has been closed with rubber stopper with 0.2 μm PTFE filter as outlet tube, Weighed and incubated at required temperature, shaking at 200 rpm. Tracking weight loss:

- For 65h fermentation procedure: weigh at 18h - 24h - 42h - 48h - 65h
- For 54h fermentation procedure: weigh at 6h - 24h - 30h - 48h - 54h

Stop fermentation:

Fermentations have been spun down at 4700 rpm, 10 min, 4°C. Supernatant has been poured over through paper filter and said filtrate has been used for measure of ethanol, glycerol, and glucose.

*Results*

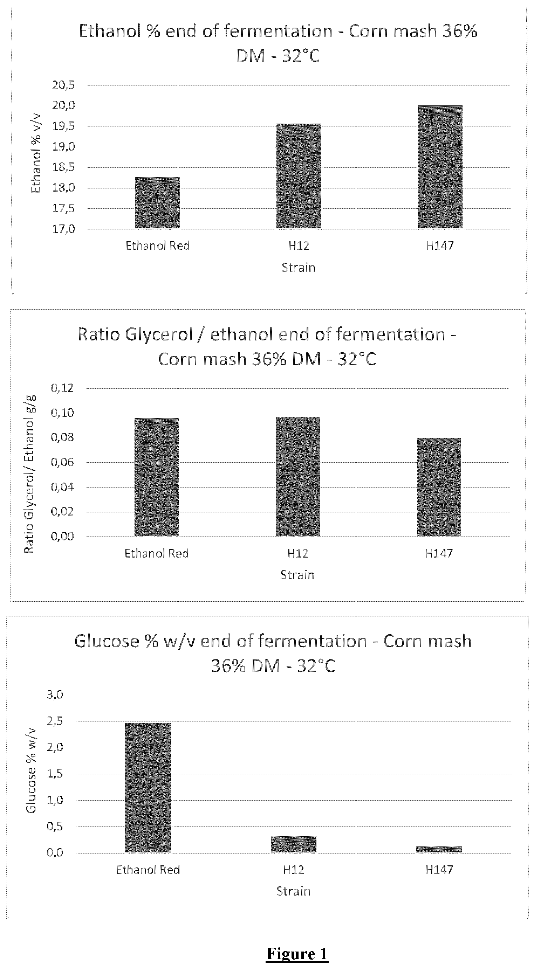

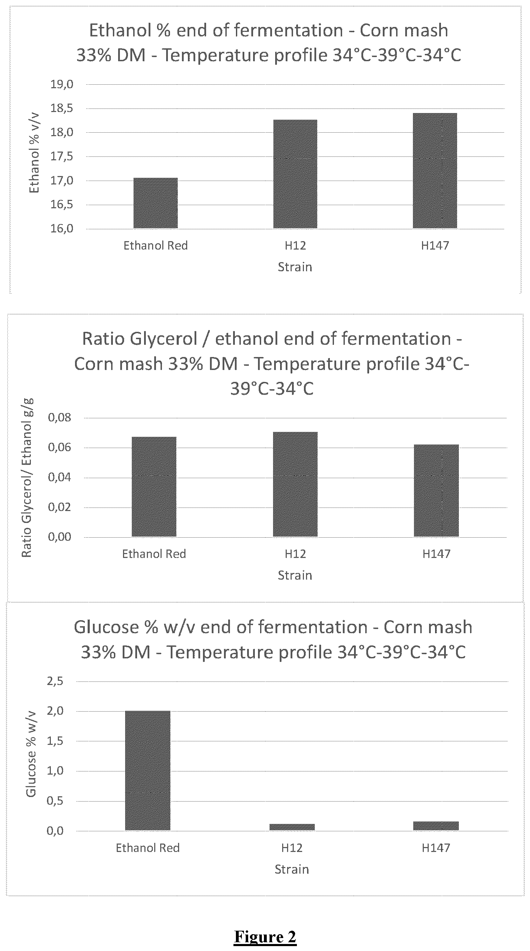

The results are presented in tables 1 and 2 below and in Figures 1 and 2.

**Table 1**

| Condition name | T (°C) | Strain | Weight loss (%w/w) | | | | | | Endpoint measurements | | | | |
|---|---|---|---|---|---|---|---|---|---|---|---|---|---|
| | | | 0h | 6h | 24 h | 30 h | 48 h | 54 h | Alco hol (% v/v) | D-gluc ose (g/L) | Glyc erol (g/l) | Glycerol/ Ethanol g/g | Gluco se % w/v |
| High solid content 36% DM | 32 | Ethanol Red | 0, 00 | 1, 20 | 9, 90 | 11, 40 | 13, 30 | 13, 50 | 18,2 6 | 24,5 9 | 14,0 1 | 0,095894 51 | 2,459 31 |
| High solid content 36% DM | 32 | H12 | 0, 00 | 0, 90 | 9, 60 | 11, 10 | 13, 90 | 14, 40 | 19,5 6 | 3,11 | 15,1 6 | 0,096888 484 | 0,311 15 |
| High solid content 36% DM | 32 | H147 | 0, 00 | 1, 00 | 9, 80 | 11, 40 | 14, 30 | 14, 60 | 20,0 0 | 1,21 | 12,7 6 | 0,079755 438 | 0,121 45 |

EP 4 786 576 A1

**Table 2**

| Condition name | T (°C) | Strain | Weight loss (%w/w) | | | | | | Endpoint measurements | | | | |
|---|---|---|---|---|---|---|---|---|---|---|---|---|---|
| | | | 0h | 18 h | 24 h | 42 h | 48 h | 65 h | Alco hol (% v/v) | D-gluc ose (g/L) | Glyc erol (g/l) | Glycerol/ Ethanol g/g | Gluco se % w/v |
| High solid content 33% DM | 34-39 | **Ethanol Red** | 0, 00 | 8, 69 | 10 ,2 3 | 12, 21 | 12, 38 | 12, 56 | 17,0 6 | 20,0 0 | 9,16 | 0,06715 0865 | 1,999 53 |
| High solid content 33% DM | 34-39 | **H12** | 0, 00 | 8, 51 | 10 ,2 3 | 12, 81 | 13, 07 | 13, 42 | 18,2 6 | 1,15 | 10,3 0 | 0,07052 615 | 0,115 445 |
| High solid content 33% DM | 34-39 | **H147** | 0, 00 | 8, 17 | 9, 80 | 12, 56 | 12, 99 | 13, 24 | 18,4 0 | 1,57 | 9,11 | 0,06190 788 | 0,157 321 |

EP 4 786 576 A1

The results obtained clearly demonstrate that strains H12 and H147:

- consume more D-glucose than the reference Ethanol Red® strain in both conditions, since the residual D-glucose concentration at the end of fermentation is close to 0;

- produce more alcohol (ethanol) than the reference Ethanol Red® strain in both conditions.

Further H147 strains produce less glycerol than the reference Ethanol Red® strain in both conditions. Glycerol being the co-product of ethanol, this indicates that the glucose consumed is preferably converted to produce ethanol.

Such results in high solid content or high gravity content allows to produce more ethanol while reducing steam consumption for the distillation stage and therefore the energy required for such step. Therefore, strains H12 and H147 make it possible to improve economic and environmental conditions by using these strains for ethanol production.

These results reveal that the fermenting organism of the invention allows to obtain

- a higher ethanol content at the end of fermentation than *Saccharomyces* strain I-4071 under same fermentation conditions;
- an increased temperature tolerance than *Saccharomyces* strain I-4071 under same fermentation conditions;
- a lower residual substrate percentage at the end of fermentation than *Saccharomyces* strain I-4071 under same fermentation conditions;
- a lower glycerol content at the end of fermentation than *Saccharomyces* strain I-4071 under same fermentation conditions; and
- a lower ratio glycerol/ethanol than *Saccharomyces* strain I-4071 under same fermentation conditions.

Hence, the present invention provides new robust yeast strains capable of improving the efficiency of ethanol production while being economical and good for the environment.

**Example 2:**

*Materials and methods*

**Strains:**

Yeast : *Saccharomyces cerevisiae*
Strains: I-6167 *(Saccharomyces cerevisiae* strain H12)

The materials and methods are the same as for example 1 except that they are conducted in a well-known fed-batch device. The person skilled in the art will know how to adapt from batch to fed-batch.

*Results*

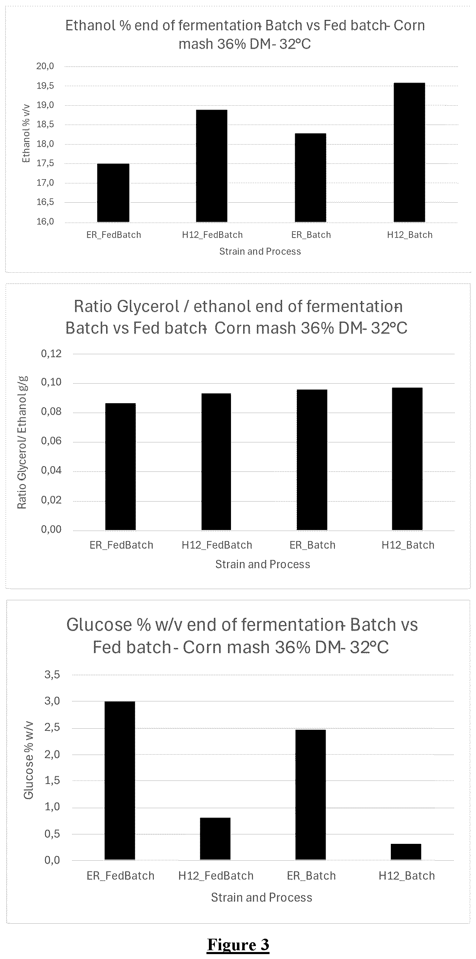

In fed and in batch regime, the H12 strain performs better than the Ethanol Red strain. This gain is reflected in the ethanol content (Figure 3). In fed batch mode, H12 shows a significative improvement compared to Ethanol Red strain.

The residual sugars quantified (Figure 3) show that Ethanol Red strain is leaving a high quantity of glucose at 65h (2.5% in batch, 3% in fed batch). This phenomenon was expected and is caused by the combination of high mash dry matter combined with high temperature (32°C). These fermentation conditions enable to differentiate the H12 strain according to the invention able to manage such a stress. The fermenting organisms of the invention thus fit this objective (glucose residual bellow 1%).

Hence, the present invention provides new robust yeast strains capable of improving the efficiency of ethanol production in industrial scale fermentation while being economical and good for the environment.

**Claims**

**1.** A fermenting organism which is a *Saccharomyces cerevisiae* strain selected from:

- *Saccharomyces cerevisiae* strain H147, which was deposited on December 20, 2024 at the CNCM under Number I-6168; and
- *Saccharomyces cerevisiae* strain H12, which was deposited on December 20, 2024 at the CNCM under Number I-6167.

2. A derivative of the fermenting organism as claimed in claim 1.

3. The fermenting organism of claim 1 or the derivative of claim 2, wherein it has one or more defining characteristics and/or fermentation characteristics selected from:

        a. a higher ethanol content at the end of fermentation than *Saccharomyces cerevisiae* strain I-4071 under the same fermentation conditions;
        b. an increased temperature tolerance than *Saccharomyces cerevisiae* strain I-4071 under the same fermentation conditions;
        c. a lower residual substrate percentage at the end of fermentation than *Saccharomyces cerevisiae* strain I-4071 under the same fermentation conditions;
        d. a lower glycerol content at the end of fermentation than *Saccharomyces cerevisiae* I-4071 under the same fermentation conditions; and
        e. a lower ratio glycerol/ethanol than *Saccharomyces cerevisiae* strain I-4071 under the same fermentation conditions.

4. The fermenting organism or the derivative of claim 3, wherein the fermentation conditions are a temperature of 32°C and a dry solid content of 36%.

5. The fermenting organism or the derivative of claim 4, wherein the fermenting organism or the derivative produces an ethanol content of at least about 18.5% v/v, preferably at least 19% v/v, more preferably et least 19.5% v/v or 20% v/v after 54 hours of fermentation.

6. The fermenting organism or the derivative of claim 3, wherein the fermentation conditions are a temperature of between 34°C and 39°C and a dry solid content of 33%.

7. The fermenting organism or the derivative of claim 6, wherein the fermenting organism or the derivative produces an ethanol content of at least about 17.5% v/v, preferably at least 18% v/v, more preferably at least 18.2% v/v or 18.3% v/v or 18.4% v/v after 65 hours of fermentation.

8. A composition comprising the fermenting organism or the derivative of claims 1 to 7 and one or more components selected from the group consisting of surfactants, emulsifiers, gums, swelling agents, protectants, antioxidants and processing aids.

9. A process for producing ethanol from a biomass comprising contacting the biomass with the fermenting organism or the derivative of claims 1 to 7 or the composition of claim 8.

10. The process of claim 9, wherein the biomass is corn mash.

11. The process of claim 9 or 10, wherein the process is batch fermentation, fed-batch fermentation or continuous fermentation.

12. The derivative of claim 2, wherein it is a hybrid produced by culturing a first yeast strain with the *Saccharomyces cerevisiae* strain of claim 1 under conditions which permit combining of DNA between the first yeast strain and the *Saccharomyces cerevisiae* strain of claim 1.

13. The derivative of claim 2, wherein it is a mutant produced by spontaneous mutation of the genome of the fermenting organism of claim 1 or a mutant induced by mutagenesis.

14. The derivative of claim 2, wherein it is a recombinant produced by genetically modifying the fermenting organism of claim 1 to express a heterologous enzyme.

15. The derivative of claim 14, wherein the heterologous enzyme is selected from the group consisting of alphaamylase, glucoamylase xylose isomerase, xylose reductase, xylitol dehydrogenase, D-xylulokinase, arabinose isomerase, ribulokinase, a ribulose 5 phosphate epimerase, and combinations thereof.

**Figure 1**

**Figure 2**

**Figure 3**

# EP 4 786 576 A1

## EUROPEAN SEARCH REPORT

**Application Number**

EP 25 30 5155

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2024/064901 A2 (NOVOZYMES AS [DK]; MICROBIOGEN PTY LTD [AU]; THOMPSON BRIANNA [US]) 28 March 2024 (2024-03-28) | 2-15 | INV. C12N1/18 C12P7/06 |
| A | * abstract * <br> * examples * <br> * claims 2,3,11,14-16 * <br> ----- | 1 | ADD. C12R1/865 |
| X | WO 2016/153924 A1 (NOVOZYMES AS [DK]; MICROBIOGEN PTY LTD [AU]; HEADMAN JENNIFER [US]) 29 September 2016 (2016-09-29) | 2-13 | |
| A | * abstract * <br> * examples 19-22, 26 * <br> * claims 5, 12-14, 19 * <br> ----- | 1 | |

TECHNICAL FIELDS SEARCHED (IPC)

C12N
C12P
C12R

The present search report has been drawn up for all claims

| Place of search | Date of completion of the search | Examiner |
|---|---|---|
| The Hague | 7 July 2025 | Sonnerat, Isabelle |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

24

## ANNEX TO THE EUROPEAN SEARCH REPORT
## ON EUROPEAN PATENT APPLICATION NO.

EP 25 30 5155

This annex lists the patent family members relating to the patent documents cited in the above-mentioned European search report.
The members are as contained in the European Patent Office EDP file on
The European Patent Office is in no way liable for these particulars which are merely given for the purpose of information.

07-07-2025

| Patent document cited in search report | | Publication date | Patent family member(s) | | Publication date |
|---|---|---|---|---|---|
| WO 2024064901 | A2 | 28-03-2024 | AU 2023347311 | A1 | 27-03-2025 |
| | | | WO 2024064901 | A2 | 28-03-2024 |
| WO 2016153924 | A1 | 29-09-2016 | AR 104098 | A1 | 28-06-2017 |
| | | | AU 2016235802 | A1 | 24-08-2017 |
| | | | US 2018105841 | A1 | 19-04-2018 |
| | | | WO 2016153924 | A1 | 29-09-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 118146965
- CN 117844664
- WO 2024123691 A
- WO 2024064901 A
- WO 2016174349 A
- WO 2015121595 A
- WO 2022216622 A
- WO 2015101753 A
- WO 2017037362 A
- WO 2020007823 A
- WO 2018138135 A
- EP 1724336 A
- US 10106823 B
- WO 2006113683 PCT
- US 20070014905 A
- WO 2006113683 A

**Non-patent literature cited in the description**

- **MOHD AZHAR SH** ; **ABDULLA R** ; **JAMBO SA** ; **MARBAWI H** ; **GANSAU JA** ; **MOHD FAIK AA** ; **RODRIGUES KF**. *Yeasts in sustainable bioethanol production: A review. Biochem Biophys Rep.*, 06 March 2017, vol. 10, 52-61
- **DA SILVA FERNANDES F** ; **DE SOUZA ÉS** ; **CARNEIRO LM** ; **ALVES SILVA JP** ; **DE SOUZA JVB** ; **DA SILVA BATISTA J**. Current Ethanol Production Requirements for the Yeast Saccharomyces cerevisiae. *Int J Microbiol.*, 13 August 2022, vol. 2022, 7878830
- **G. REED** ; **T.W. NAGODAWITHANA**. Yeast Technology. Van Nostrand Reinhold
- Saccharomyces cerevisiae. *CNCM*, 20 December 2024 (I-6168)
- *Saccharomyces cerevisiae strain Ethanol Red*, 04 September 2008
- **AUSUBEL, F. M. et al.** Current Protocols in Molecular Biology. ohn Willey & Sons Inc, 1997, vol. 2, 1-5
- Sporulation and Hybridisation of yeast. **R.R. FOWELL**. The Yeasts. Academic Press, 1969, vol. 1
- **SPENCER et al.** Yeast Technology. Springer Verlag, 1990
- **INGE-VECHYMOV et al.** *Genetika*, 1986, vol. 22, 2625-2636
- **JOHNSTON**. Yeast technology. Springer Verlag, 1990
- **LAWRENCE C.W.** *Methods in Enzymology*, 1991, vol. 194, 273-281
- **AUSUBEL, F. M. et al.** Current Protocols in Molecular Biology. John Wiley & Sons Inc, 1997, vol. 2, 1-7
- **G. REED** ; **T. W. NAGODAWITHANA**. Yeast Technology. Van Nostrand Reinhold, 1991
- **THOMAS et al.** *Journal of Applied Microbiology*, 2001, vol. 90, 819-828